# EUROPEAN PATENT APPLICATION

(11) **EP 1 356 860 A2**
(43) Date of publication of application: **29.10.2003**
(21) Application number: 02023726.9
(22) Date of filing: 23.10.2002
(51) Int. Cl.: B01J 19/00

(54) **Quality control method of DNA microarray**

(30) Priority: 07.03.2002 KR 2002012158
(71) Applicant: SAMSUNG ELECTRONICS CO., LTD., Suwon-City, Kyungki-do (KR)
(72) Inventor: Cho, Jun-hyeong, Yuseong-gu, Daejeon (KR); Huh, Nam, Seoul (KR); Shim, Jeo-young, Masan-si, Gyeongsangnam-do (KR); Kim, Kyeong-hee, Daejeon (KR); Park, Ga-young, Samsung Advanced Inst. of Techn., Yongin-si, Gyeonggi-do (KR)
(74) Representative: Grünecker, Kinkeldey, Stockmair & Schwanhäusser Anwaltssozietät

(57) **Abstract**

A quality control method of a DNA microarray is provided which includes preparing a DNA spotting solution containing a first fluorescent dye having particular excitation and emission wavelengths, applying the DNA spotting solution to a substrate of a DNA chip to form DNA spots in which the first fluorescent dye is bound to the substrate, and detecting fluorescent signals from the DNA spots. In the DNA microarray quality control method, since a signal from a fluorescent dye covalently bound to the solid chip surface together with DNA probe is used for the quality control, staining and dye-removing processes, which were performed in conventional quality control methods, are unnecessary.

## Description

### BACKGROUND OF THE INVENTION

### 1. Field of the Invention

The present invention relates to a DNA microarray, and more particularly, to a quality control method of a DNA chip.

### 2. Description of the Related Art

A DNA microarray refers to a DNA chip manufactured by immobilizing oligonucleotide probes, each probe having a known sequence of a few to hundreds of nucleotides, at hundreds to hundreds of thousands of appropriate positions on a solid surface made of, for example, silicon, surface-modified glass, polypropylene, or activated polyacrylamide. As a target DNA to be assayed is applied to the DNA chip, a fragment of the target DNA complementarily hybridizes to the oligonucleotide probes immobilized on the DNA chip. The hybridization is optically or radiochemically detected and analyzed to identify the nucleotide sequence of the target DNA, which is called sequencing by hybridization (SBH).

Use of the DNA chip manufactured as a microarray reduces the size of a DNA assay system and enables genetic assay with a trace of a sample. In addition, multiple sequences of a target DNA can be simultaneously assayed, thereby rapidly providing the genetic information of the target DNA at low costs. The DNA microarray chip can assay a large amount of genetic information within a short period of time and the relevancy of the genes. Accordingly, the DNA chip is expected to have wide applications, for example, to genetic disorder and cancer diagnosis, mutant and pathogen detection, gene expression assay, drug discovery, etc. In addition, the DNA chip can be used as a microorganism or pollutant detector to find out antidotal genes and further to produce antidotes on a large scale based on genetic recombination technologies. The DNA chip can lead to great improvements in most biological industries, including the production of medicinal crops or low-fat meat.

DNA microarrays are classified into an oligo-chip and a cDNA chip according to the type of probes immobilized thereon. According to the manufacturing method, DNA microarrays are classified into a lithography chip, a pin-type spotting chip, and an ink-jet type spotting chip. A DNA microarray is manufactured through diversified and complex chemical processes according to the type of immobilized probes. Since a trace of a DNA probe is immobilized in a very small area on a glass surface, quantitative spot-to-spot and chip-to-chip variations of probes are considerably great, and thus the results from the hybridization are inconsistent.

To address this problem, a quality control technique of a DNA chip before hybridization has been suggested. *Brown et al.(* "Quantitative Monitoring of Gene Expression Patterns with a Complimentary DNA Microarray" M. Schena, D. Shalon, R.W. Davis and P.O. Brown. Science 270:467-470 (1995).) developed a method for inspecting DNA probe spot uniformity and glass surface damage by laser scanning a light scattering due to salts present in the DNA probe spots immobilized on the glass surface. This method can be conveniently applied only immediately after DNA probe spotting but is limited after completion of the DNA chip manufacturing because the salts are removed from the DNA probe spots through immobilization and washing processes following the spotting.

Recently, a method for staining DNA spots on a glass surface using a fluorescent material capable of binding to the DNA probes was developed *(Battaglia C,* Salani G, *Consolandi C, Rossi* Bernardi *L, De Bellis* G., Analysis of DNA Microarrays by Non-destructive Fluorescent Staining Using SYBR Green II, Biotechniques, 2000 July, 29:78-81). According to this method, the DNA probes immobilized on the glass surface of the DNA chip are treated with fluorescent SYBR Green II having a high affinity to a single stranded DNA (ssDNA) and detected by laser scanning. The fluorescent material bound to the DNA probes was reported to be completely removed by washing. Compared with the Brown's method, the fluorescent staining method can be applied to inspect the DNA microarray after the completion of the chip manufacturing, but needs a number of washing processes for staining and dye-removing. Unfortunately, the fluorescent material is likely to remain on the glass surface after the washing and thus affects the subsequent hybridization process.

### SUMMARY OF THE INVENTION

Accordingly, the present invention provides a convenient, non-destructive method for controlling the quality and size of DNA spots on a DNA microarray, which does not need staining and dye-removing processes.

In one aspect, the quality control method of the DNA chip according to the present invention comprises: preparing a DNA spotting solution containing a fluorescent dye; applying the DNA spotting solution to a substrate as spots to manufacture the DNA chip; and detecting fluorescent signals from the DNA spots.

### BRIEF DESCRIPTION OF THE DRAWINGS

The above object and advantages of the present invention will become more apparent by describing in detail exemplary embodiments thereof with reference to the attached drawings in which:
FIG. 1 shows the results of scanning oligonucleotide probe spots immobilized on a DNA microarray before (QC Test) and after (Use Test) hybridization to a target DNA, performed in Example 1; and
FIG. 2 shows the results of scanning cDNA probe spots immobilized on a DNA microarray before (QC Test) and after (Use Test) hybridization to a target DNA, performed in Example 2.

### DETAILED DESCRIPTION OF THE INVENTION

Unlike conventional DNA chip quality control methods in which DNA probes are spotted on a DNA chip substrate and then a fluorescence dye is bound to the DNA probe, in a DNA chip quality control method according to the present invention, a DNA spotting solution to be applied to the DNA chip substrate is prepared with a fluorescent dye, and the fluorescent dye is covalently bound to the substrate surface while DNA probes are immobilized on the DNA chip substrate. After completion of the DNA chip manufacturing, fluorescence from the DNA spots is scanned to non-destructively determine DNA spot uniformity.

Uniformity of fluorescent signals from the DNA probe spots is determined by the diameter and shape of the DNA probe spots and the intensity of the fluorescence.

The fluorescent dye used in the present invention has a dye group (F) for generating a fluorescent signal and a functional group (R) that covalently binds to a solid substrate, which is expressed as follows:

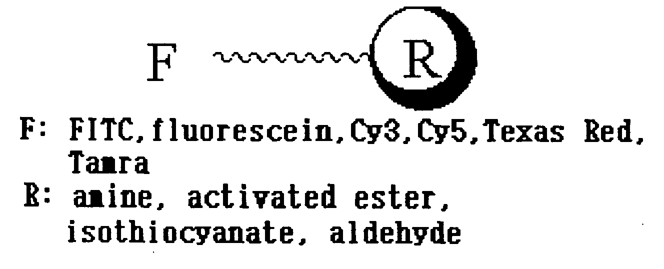

Suitable dye groups for generating the fluorescent signal have no spectral interference with a dye for target DNA labelling, used to detect hybridization after the manufacture of the DNA chip, and include fluorescein isothiocyanate (FIFC), fluorescein, Cy3, Cy5, Texas Red, N,N,N',N'-tetramethyl-6-carboxyrhodamine (TAMRA), etc., which have no spectral interference.

The functional group that binds to the solid substrate should be capable of covalently binding to a functional group on the solid substrate surface, such as amine, aldehyde, or poly-lysine, according to the type of the DNA microarray. Suitable functional groups of the fluorescent dye include amine, isothiocyanate (-NCS), activated ester, and aldehyde.

The quality control method of the DNA spots on the DNA chip manufactured as described above can be modified according to the type of the fluorescent dye used. For example, when the dye group of the fluorescent dye is FIFC or fluorescein, the dye group is excited at 488 nm wave length by an argon ion laser, and a fluorescent signal is analysed using a 520-nm wave length filter.

The quality control method of the DNA microarray according to the present invention can be applied to another type of microarray, such as an RNA chip or a protein chip.

The present invention will be described in greater detail with reference to the following examples. The following examples are for illustrative purposes and are not intended to limit the scope of the invention. In the following examples, it was experimentally determined whether or not a fluorescent dye immobilized on a substrate together with DNA probes in the manufacture of a DNA chip affects a fluorescent intensity from DNA spots after hybridization of the DNA probes with a fluorescent-labelled target DNA on the DNA chip. Two types of DNA chips, an oligonucleotide probe-immobilized DNA chip and a cDNA-immobilized DNA chip, were used.

### Example 1: Spot quality control in oligonucleotide probe-immobilized DNA chip

### A. Manufacture of DNA-oligonucleotide chip using gel matrix

Two types of oligonucleotide probes (perfect match (PM)-5'-TGTAGACACGCACCTCCGTG-3'; or mismatch (MM) - 5'-TGTAGACACCCACCTCCGTG-3') and 4'-(aminomethyl)fluorescein (0 µM, 1 µM, and 2 µM) as a fluorescent dye were added to a gel matrix solution with stirring and left at 37°C for 14 hours to form matrix-DNA conjugates. The resultant solution was used as a spotting solution. The spotting solution was spotted on a glass surface, which had been treated to expose amine groups, and left in a wet chamber at 37°C for 4 hours. Next, a region of the glass surface to which the spotting solution was not applied was treated to negatively charge amine groups of the region and thus to prevent the target DNA from adhering to the non-spotting region, which is a process necessary for background noise control. In this regard, 5 g of succinic anhydride was dissolved in 315 mL of 1-methyl-2-pyrrolidone (NMP), and 35 ml of sodium borate was added to the solution with stirring. The glass surface was treated with the solution, washed with distilled water and left in a dryer.

The 4-(aminomethyl)fluorescein used as the fluorescent material has the following formula:

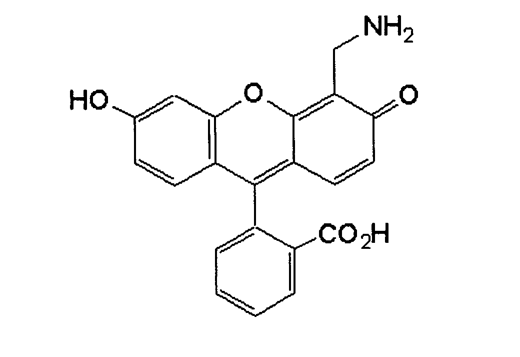

### B. Measurement of hybridization sensitivity by the flourescence of Cy3

A fluorescent signal from the fluorescent-labelled target material hybridized to the probes on the DNA microarray was detected using a scanner (ScanArray Scanner, GSI Lunonics) on a 10-µm-pixel resolution. The intensity of Spots were produced using GenePix software.

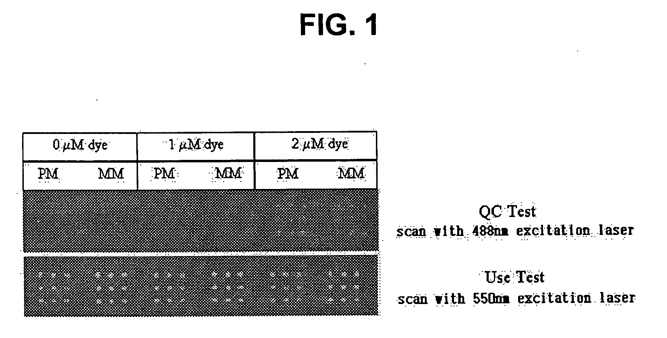

The result of scanning the DNA microarray spots before hybridization to the target DNA (Quality Control (QC) Test), which was prepared using the gel matrix as described above, and the result of scanning the DNA microarray spots after the hybridization to the target DNA (Use Test) are shown in FIG. 1.

In scanning the DNA spots for the QC Test, the DNA spots were excited by scanning with an argon ion laser at 488 nm, and a fluorescent emission signal was obtained using a 520 nm wave length filter. In the Use Test, the the target DNA, which is hybridezed to the DNA spots was excited at 550 nm, and a fluorescent emission signal was obtained using 570 nm wave length filter. In the Use Test, a sequence of 20 nucleotides with nucleotide C (cytocine) base corresponding to codon 264 in exon 4 of the hepatocyte nuclear factor (HNF)-1 α gene in the middle of the sequence and Cy3 attached to its 5'-terminal, which is expressed as (5'-Cy3-CACGGAGGTGCGTGTCTACA-3'), was used as the target DNA.

As shown in FIG. 1, 9 spots for each of the perfect-match and mismatch probes were spotted. The spot diameter was 170 ± 15 µm, and the spot interval was adjusted to 375 µm.

### C. Experimental results

As shown in FIG. 1, in use test the fluorescent intensity of the probe spots immobilized together with 4'-(aminomethyl)fluorescein was not significantly different from that of dye-free probe spots. Evidently, the hybridization of the target DNA to the probe spots is unaffected by the use of the 4'-(aminomethyl)fluorescein.

**Table 1.**

| Fluorescent intensity of probe spots on the DNA microarray using a gel matrix in the QC Test and Use Test | | | | |
|---|---|---|---|---|
| 4'-(aminomethyl)fluorescein | QC Test | | Use Test | |
| | PM | MM | PM | MM |
| O µM | 538 | 458 | 36034 | 18438 |
| 1 µM | 770 | 699 | 35153 | 17545 |
| 2µM | 1306 | 1178 | 34765 | 18661 |

### Example 2: Spot quality control in cDNA chip

### A. Probe gene amplification through polymerase chain reaction (PCR)

A recombinant gene capable of producing glyceraldehyde-3-phosphate dehydrogenase (GAPDH) was isolated using a DNA extraction kit (GAIGEN). The isolated recombinant GAPDH gene was selectively amplified through PCR in a reaction solution having a final volume of 100 µL.

50 g of the pure recombinant GAPDH DNA, 2.5 U of thermally stable DNA polymerase, 200 µM of each dNTP (dATP, dTTP, dGTP, and dCTP), 50 mM Tris-HCI, 40 mM KCI, 1.5 mM MgCl₂, and 100 pmols of each of a sense primer (5'-CTGGTAAGTTTAGTCTTTTTGTC-3') and an anti-sense primer (5'-CAGTGCCAAGCTTGCATGCCT-3') were used. Thirty five cycles of PCR, one cycle including denaturation at 94°C for 30 seconds, annealing at 55°C for 60 seconds, and extension at 72°C for 90 seconds, were carried out. Pre-denaturation at 94°C and last-extension at 72°C were performed for 5 minutes each. The amplicon was purified using the DNA extraction kit (GAIGEN) and used as probes to be immobilized on a glass substrate.

### B. Amplified GAPDH gene spotting: Manufacture of cDNA microarray

The amplified GAPDH gene was mixed to a final concentration of 0.25 mg/mL with 50% DMSO in two separate tubes. To one of the tubes, FITC dye was added to a final concentration of 8 µM. The amplified gene, DNSO, and FITC dye in each of the tubes were thoroughly mixed by Voltex mixer. The reagent mixtures were transferred into 384-well plates for spotting. The spotting solution was spotted on a glass substrate, which had been treated to have amine groups, in a 20X4 array, and heated at 80°C for 4 hours. Next, a region of the glass surface to which the spotting solution was not applied was treated to negatively charge amine groups of the region and thus to prevent the target DNA from adhering to the non-spotting region, which is a process necessary for controlling background noise. In this regard, 5 g succinic anhydride was dissolved in 315 mL of 1-methyl-2-pyrrolidone_ (NMP), and 35 ml of sodium borate was added to the solution with stirring. The glass surface was treated with the solution, washed with distilled water and left in a dryer.

The FITC used as the fluorescent dye has the following formula:

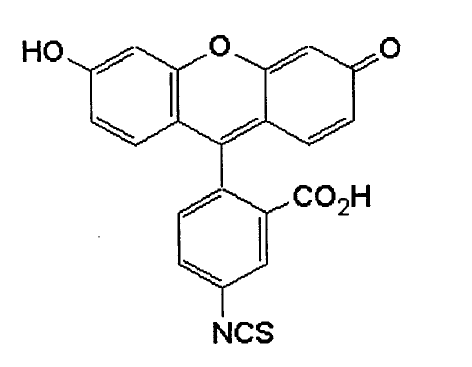

### C. Preparation of fluorescent-labelled target DNA by PCR

The GAPHD producing recombinant gene isolated using the DNA extraction in the above experiment was used to prepare a fluorescent-labelled target DNA. The isolated recombinant GAPDH was selectively amplified through PCR in a reaction solution having a final volume of 50 µL.

50 g of the pure recombinant GAPDH DNA, 2.5 U of thermally stable DNA polymerase, 200 µM of each dNTP (dATP, dGTP, and dCTP), 65 µM dTTP, 130 µM Cy3-dUTP, 50 mM Tris-HCI, 40 mM KCI (pH 8,3), 1.5 mM MgCl₂, and 100 pmols of each of a sense primer (5'-CTGGTAAGTTTAGTCTTTTTGTC-3') and an anti-sense primer (5'-CAGTGCCAAGCTTGCATGCCT-3') were used. PCR was carried out in the same conditions as in the above experiment. The amplicon was purified using the DNA extraction kit (GAIGEN), and its concentration was measured by spectrophotometry. For hybridization efficiency, the concentration of the purified GAPDH DNA was constantly adjusted to 50 ng/µL. The target DNA of the GAPDH gene was fluorescently labelled with Cy3 during the PCR with the addition of Cy3-dUTP and included 600 basepairs.

### D. Fluorescent-labelled target DNA hybridization to cDNA microarray

The cDNA chip manufactured above was preliminarily hybridized at 50°C for 45 minutes in a solution of 4X SSC (standard saline-citrate), 0.1% SDS (sodium dodecyl sulfate), and 10 mg/mL BSA (bovine serum albumin) and washed with isopropanol and pure water. Water was removed from the cDNA chip by centrifugation at 500 rpm for 3 minutes.

1 µg of the fluorescent-labelled target DNA prepared in the above experiment was denatured at 95°C for 5 minutes, left in ice for 3 minutes, mixed with a solution of 3X SSC, 0.1% SDS, 0.5 mg/mL yeast tRNA, and 30 µg/mL herring sperm DNA, and dropped on the cDNA chip. The cDNA chip was covered with a cover glass to allow wide spreading of the solution and hybridized overnight at 50°C. The chip substrate was washed once for 5 minutes with a solution of 0.1X SSC (1.5 mM sodium citrate, 15 mM NaCI, pH 7.0) and 0.1% SDS, and twice for 5 minutes with 0.1X SSC at 25°C. Next, water was removed from the chip substrate by centrifugation in the same conditions as the above.

### E. Measurement of hybridization sensitivity by the fluorescence of Cy3

Hybridization sensitivity was measured in the same manner as in Example 1.

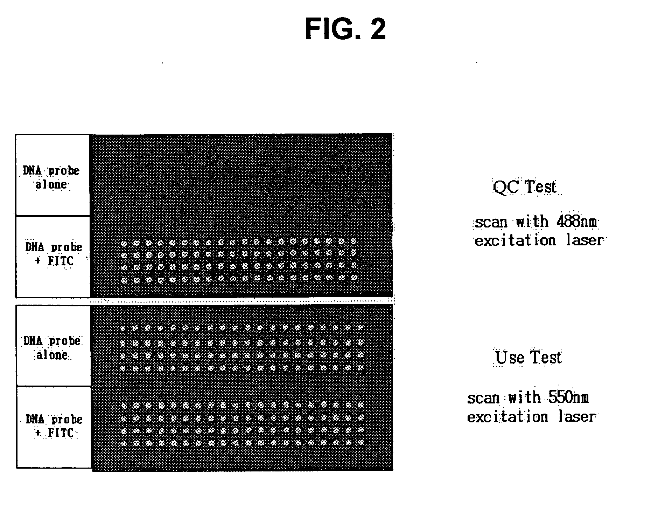

The result of scanning the cDNA microarray spots before the hybridization to the target DNA (QC Test), performed as described above, and the result of scanning the cDNA microarray spots after the hybridization to the target DNA (Use Test) are shown in FIG. 2.

In scanning the DNA probe spots for the QC Test, the DNA probe spots were excited by scanning with an argon ion laser at 488 nm, and a fluorescent emission signal was obtained using a 520 nm wave length filter. In the Use Test, the target DNA, which is hybridized to the DNA probe spots, was excited at 550nm, and a fluorescent emission signal was obtained using a 570 nm wave length filter.

As shown in FIG. 2, 80 spots were spotted on each DNA chip. The spot diameter was 170 ±15 µm, and the spot interval was adjusted to 375 µm.

### F. Experimental Results

Based on the results of FIG. 2, the fluorescent intensity was compared in two cases, i.e., when FITC dye was used and when no fluorescent dye was used, a shown in Table 2 below.

As shown in Table 2, the fluorescent intensity of the probe spots immobilized together with FITC was not significantly different from that of dye-free probe spots. Evidently, the hybridization of the target DNA to the probe spots is unaffected by the use of the FITC.

**Table 2.**

| Fluorescent intensity of probe spots on the cDNA microarray using GAPDH amplicon in the QC Test and Use Test | | | | |
|---|---|---|---|---|
| | QC Test | | Use Test | |
| | Spot Diameter | Intensity | Spot Diameter | Intensity |
| DNA probe | - | - | 148 | 9359 |
| DNA probe +FITC | 165 | 18905 | 150 | 9852 |

As described above, in the quality control method of a DNA microarray according to the present invention, a signal from a fluorescent dye covalently immobilized on the solid surface of the DNA chip together with DNA probe is detected and used for the quality control of the DNA microarray. Compared with conventional quality control methods involving a separate staining process, there is no need to perform staining and dye-removing processes.

In addition, the fluorescent dye covalently bound to the solid substrate of the DNA chip together with DNA probes hardly affects the fluorescent intensity of the DNA spots after hybridization to a target DNA, thereby eliminating the likelihood of the fluorescent dye affecting the hybridization in conventional methods.

The DNA microarray manufactured by the above-describe method can be quality-controlled without a staining process and provides a reliable, consistent result of hybridization.

Unlike conventional quality control methods, since the fluorescent dye is covalently bound to reactive groups on the glass chip surface together with DNA probes, the intensity of a fluorescent signal is unaffected by the sequence and length of the DNA probes, thereby enabling an accurate quality comparison of the separate DNA probe spots.

While this invention has been particularly shown and described with reference to preferred embodiments thereof, it will be understood by those skilled in the art that various changes in form and details may be made therein without departing from the spirit and scope of the invention as defined by the appended claims.

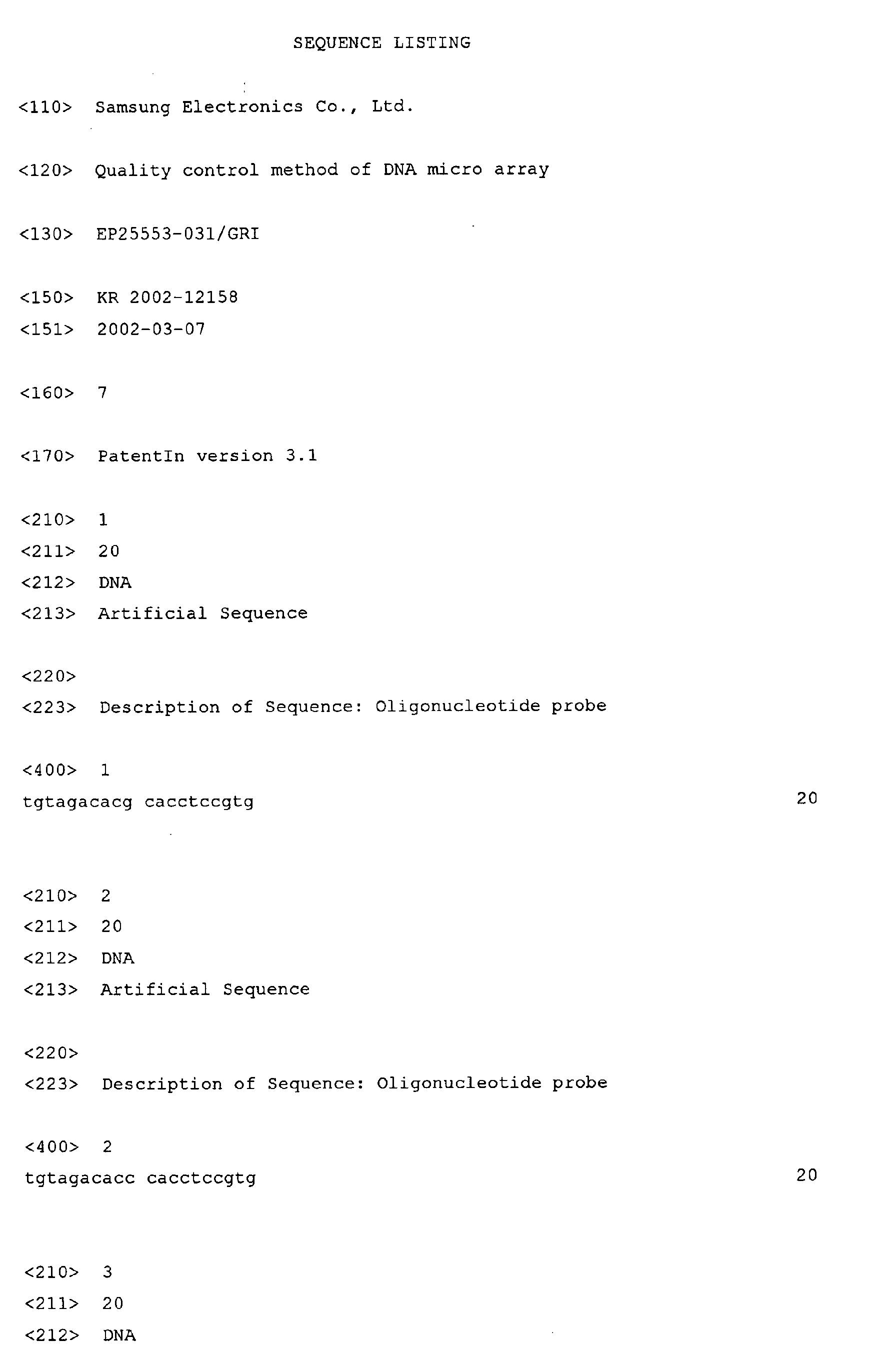

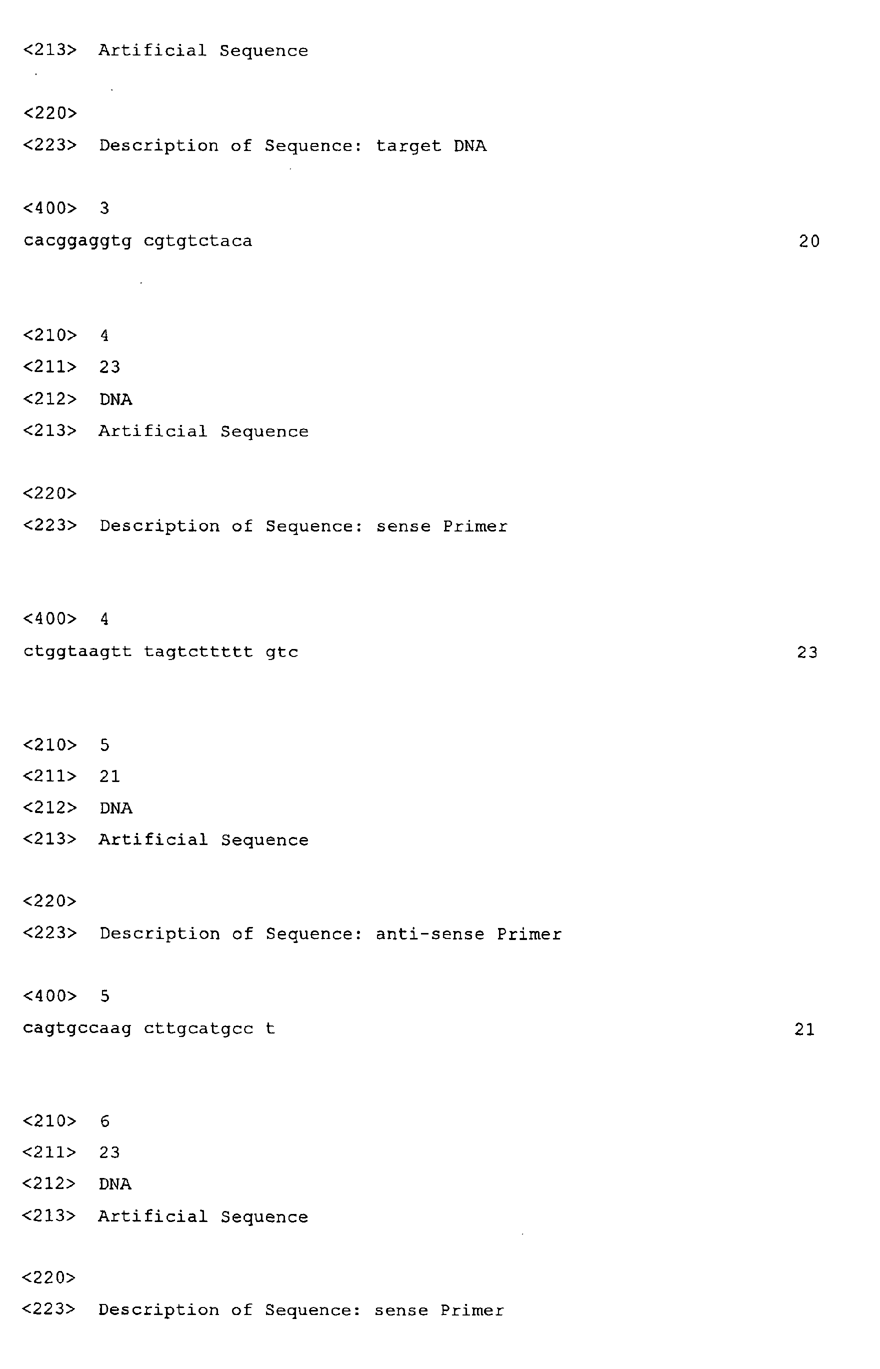

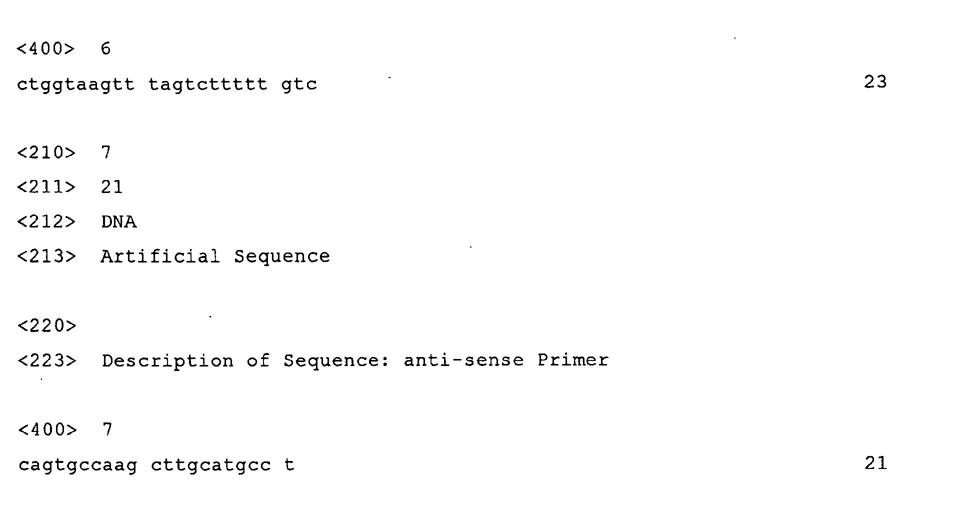

## Claims

1. A quality control method of a DNA chip, comprising:
preparing a DNA spotting solution containing a DNA probe and a first fluorescent dye having specific excitation and emission wavelengths;
applying the DNA spotting solution to a substrate of a DNA chip to form DNA spots in which the first fluorescent dye is bound to the substrate, and
detecting fluorescent signals from the DNA spots.

2. The quality control method of claim 1, wherein the first fluorescent dye has no spectral interference with excitation and emission wavelengths of a second fluorescent dye for labeling a target DNA.

3. The quality control method of claim 1, further comprising measuring uniformity of the detected fluorescent signals.

4. The quality control method of claim 3, wherein the uniformity of the detected fluorescent signals is measured by a diameter and shape of the DNA spots and a fluorescent intensity of the fluorescent signals.

5. The quality control method of claim 1, wherein the first fluorescent dye includes fluorescein isothiocyanate (FITC), fluorescein, Cy3, Cy5, Texas Red, and N,N,N',N'-tetramethyl-6-carboxyrhodamine (TAMRA).
